# EUROPÄISCHE PATENTANMELDUNG

(11) **EP 3 769 943 A1**
(43) Veröffentlichungstag der Anmeldung: **27.01.2021**
(21) Anmeldenummer: 19187559.0
(22) Anmeldetag: 22.07.2019
(51) Int. Cl.: B29C 67/24, B29D 23/20, B65D 35/22, B29L 23/00

(54) **BEHÄLTER ZUM ERZEUGEN EINER ZAHNMEDIZINISCHEN KUNSTSTOFFMASSE**

(71) Anmelder: Ivoclar Vivadent AG, 9494 Schaan (LI)
(72) Erfinder: Senti, Theresa, 9486 Schaanwald (LI); Gebhardt, Benjamin, 9472 Grabs (CH); Lerch, Stefan, 3006 Bern (CH); Lenz, Stephan, 6800 Feldkirch (AT)
(74) Vertreter: Baldus, Oliver

(57) **Zusammenfassung**

Die vorliegende Erfindung betrifft einen Behälter (100) zum Erzeugen einer selbsthärtenden oder dualhärtenden zahnmedizinischen Kunststoffmasse (200), der eine verformbare Wandung (101) aufweist, mit einer ersten Kunststoffkomponente (103-1); und einer zweiten Kunststoffkomponente (103-2) zum Härten der Kunststoffmasse (200) oder Starten einer Härtungsreaktion.

## Beschreibung

Die vorliegende Erfindung betrifft einen Behälter zum Erzeugen einer selbsthärtenden oder dualhärtenden zahnmedizinischen Kunststoffmasse und ein Verfahren zum Erzeugen einer selbsthärtenden oder dualhärtenden, zahnmedizinischen Kunststoffmasse.

Im zahnmedizinischen Bereich ist es schwierig, kleine Mengen an zahnmedizinischer Kunststoffmasse herzustellen, die aus zwei unterschiedlichen Kunststoffkomponenten zusammengemischt werden. Werden die Kunststoffkomponenten beispielsweise manuell aus unterschiedlichen Tuben entnommen und zusammengemischt, so ist es schwierig das richtige Mischungsverhältnis einzuhalten. Daneben sollte eine Homogenität der Mischung gewährleistet sein. Außerdem spielt der zeitliche Ablauf eine Rolle. Mischt ein Anwender die Kunststoffkomponenten nicht schnell genug, härtet die Kunststoffmasse aus bevor die gewünschte Mischqualität erreicht ist. Daneben ist das Einbringen von Luft in die Mischung ein Risiko.

Es ist daher die technische Aufgabe der vorliegenden Erfindung, eine Herstellung einer zahnmedizinischen Kunststoffmasse zu erleichtern.

Diese Aufgabe wird durch Gegenstände nach den unabhängigen Ansprüchen gelöst. Vorteilhafte Ausführungsformen sind Gegenstand der abhängigen Ansprüche, der Beschreibung und der Figuren.

Gemäß einem ersten Aspekt wird diese Aufgabe durch einen Behälter zum Erzeugen einer selbsthärtenden oder dualhärtenden zahnmedizinischen Kunststoffmasse gelöst, der eine verformbare Wandung aufweist, mit einer ersten Kunststoffkomponente; und einer zweiten Kunststoffkomponente zum Härten der Kunststoffmasse oder Starten einer Härtungsreaktion. Die erste Kunststoffkomponente und die zweite Kunststoffkomponente sind unvermischt in verschiedenen Volumenbereichen in dem Behälter angeordnet. Die zahnmedizinische Kunststoffmasse kann beispielsweise bei einer Behandlung eines Patienten verwendet werden. Der Behälter kann ein Einwegbehälter oder ein Mehrwegbehälter sein. Der Behälter ist derart verformbar, dass die beiden Kunststoffkomponenten durch ein Kneten des Behälters vermischt werden können.

In einem Knetvorgang wird die verformbare Wandung des Behälters deformiert, so dass auf die beiden Kunststoffkomponenten eine mechanische Kraft ausgeübt werden kann und sich diese miteinander vermischen. Dadurch entsteht im Inneren des Behälters die zahnmedizinische Kunststoffmasse, die anschließend direkt verarbeitet werden kann. Die Kunststoffmasse dient beispielsweise zum Füllen eines Zahnes oder zum Aufkleben einer Krone. Durch den Behälter wird der technische Vorteil erreicht, dass die beiden Kunststoffkomponente bereits im richtigen Mischungsverhältnis im Behälter vordosiert sein können und sich auch kleine Mengen der zahnmedizinischen Kunststoffmasse einfach und schnell herstellen lassen.

In einer technisch vorteilhaften Ausführungsform des Behälters ist der Behälter schlauchförmig oder beutelförmig. Der Beutel kann vorgefertigt geschlossen sein oder aus einer oder zwei Folien und nur durch Klemmen temporär zu einem geschlossenen Behälter werden. Dadurch wird beispielsweise der technische Vorteil erreicht, dass sich die Kunststoffkomponenten schnell und homogen miteinander vermischen lassen.

In einer weiteren technisch vorteilhaften Ausführungsform des Behälters ist die verformbare Wandung aus Silikon gebildet. Dadurch wird beispielsweise der technische Vorteil erreicht, dass der Behälter besonders gut kneten lässt, um die beiden Kunststoffkomponenten zu vermischen.

In einer weiteren technisch vorteilhaften Ausführungsform des Behälters ist die verformbare Wandung in einem Wellenlängenbereich von 390 nm bis 520 nm transparent. Dadurch wird beispielsweise der technische Vorteil erreicht, dass der Vermischungsgrad der Kunststoffkomponenten optisch erfasst werden kann.

In einer weiteren technisch vorteilhaften Ausführungsform des Behälters sind die erste Kunststoffkomponente und/oder die zweite Kunststoffkomponente strangförmig innerhalb des Behälters angeordnet. Dadurch wird beispielsweise der technische Vorteil erreicht, dass sich die beiden Kunststoffkomponenten über die Stranglänge homogen durch Kneten vermischen lassen.

In einer weiteren technisch vorteilhaften Ausführungsform des Behälters ist der Behälter innen oder außen mit einer dreidimensionalen Struktur versehen, die das Mischen des Materials unterstützt und/oder das Herausquellen der Kunststoffmasse während dem Mischvorgang verhindert.

In einer weiteren technisch vorteilhaften Ausführungsform des Behälters ist der Behälter so gestaltet, dass dieser umstülpbar ist, so dass die Außenflächen nach Innen und die Innenflächen nach außen gewendet werden. Dadurch wird beispielsweise der technische Vorteil erreicht, dass das Dosieren der Kunststoffkomponenten und/oder das Entfernen der gemischten Kunststoffmasse erleichtert werden.

In einer weiteren technisch vorteilhaften Ausführungsform des Behälters sind die strangförmige, erste Kunststoffkomponente und die strangförmige, zweite Kunststoffkomponente miteinander verflochten sind. Dadurch wird beispielsweise der technische Vorteil erreicht, dass sich die beiden Kunststoffkomponenten noch besser und einfacher vermischen lassen.

In einer weiteren technisch vorteilhaften Ausführungsform des Behälters umfasst die erste Kunststoffkomponente eine polymerisierbare organische Matrix mit einer Mischung aus Monomeren, Initiator-Komponenten, Stabilisatoren und Pigmenten umfasst.

In einer weiteren technisch vorteilhaften Ausführungsform des Behälters sind die erste und/oder zweite Kunststoffkomponente pulverförmig, pastenartig oder flüssig.

In einer weiteren technisch vorteilhaften Ausführungsform des Behälters weist die Kunststoffmasse aus der vermischten ersten Kunststoffkomponente und zweiten Kunststoffkomponente eine Viskosität auf, die für fließfähige Kunststoffmassen im Bereich von als 50 - 3000 Pas, und vorzugsweise für stopfbare Kunststoffmassen zwischen 3000 - 50.000 Pas ist. Dadurch wird beispielsweise der technische Vorteil erreicht, dass die erzeugte Kunststoffmasse gut verarbeiten lässt.

Die Messung der Viskosität von fließfähigen und stopfbaren Kunststoffkomponenten erfolgt mit einem Rheometer im Oszillationsmodus. Die Messungen werden bei Raumtemperatur (23.0 °C) durchgeführt. Die Messungen werden mit PP-MS (Platte-Platte Mess-Systemen) durchgeführt. Für stopfbare Kunststoffkomponenten wird ein Plattendurchmesser von 10 mm verwendet, für fließfähige ein Plattendurchmesser von 25 mm. Der Spaltabstand liegt je nach Füllstoffzusammensetzung zwischen 0.500 und 1.000 mm.

In einer weiteren technisch vorteilhaften Ausführungsform des Behälters umfasst der Behälter eine Tülle zum Ausdrücken der Kunststoffmasse. Dadurch wird beispielsweise der technische Vorteil erreicht, dass sich die erzeugte Kunststoffmasse gut aus dem Behälter herausdrücken und an dem gewünschten Ort applizieren lässt.

Gemäß einem zweiten Aspekt wird diese Aufgabe durch ein Verfahren zum Erzeugen einer selbsthärtenden oder dualhärtenden zahnmedizinischen Kunststoffmasse gelöst, mit dem Schritt eines Kneten eines Behälters mit einer verformbaren Wandung, der eine erste Kunststoffkomponente und eine zweite Kunststoffkomponente zum Härten der Kunststoffmasse oder Starten einer Härtungsreaktion umfasst. Durch das Verfahren werden die gleichen technischen Vorteile wie durch den Behälter nach dem ersten Aspekt erreicht.

In einer vorteilhaften Ausführungsform des Verfahrens wird der Behälter vor oder während dem Schritt des Knetens auf eine vorgegebene Temperatur erwärmt. Dadurch wird beispielsweise der technische Vorteil erreicht, dass die Vermischung der beiden Kunststoffkomponenten beschleunigt wird, z.B. durch Erniedrigung der Viskosität.

In einer vorteilhaften Ausführungsform des Verfahrens wird das Kneten oder Mischen der ersten und zweiten Kunststoffkomponente bei einem Unterdruck oder unter Vakuum durchgeführt. Dabei kann nur der Behälter unter Unterdruck oder Vakuum stehen oder die gesamte Umgebung innerhalb der Mischkammer.

Ausführungsbeispiele der Erfindung sind in den Zeichnungen dargestellt und werden im Folgenden näher beschrieben.

Es zeigen:
- Fig. 1: eine schematische Darstellung eines Behälters zum Erzeugen einer selbsthärtenden oder dualhärtenden zahnmedizinischen Kunststoffmasse; und
- Fig. 2: eine weitere schematische Darstellung des Behälters zum Erzeugen einer selbsthärtenden oder dualhärtenden zahnmedizinischen Kunststoffmasse; und
- Fig. 3: ein Blockdiagramm eines Verfahrens zum Erzeugen einer selbsthärtenden, zahnmedizinischen Kunststoffmasse.

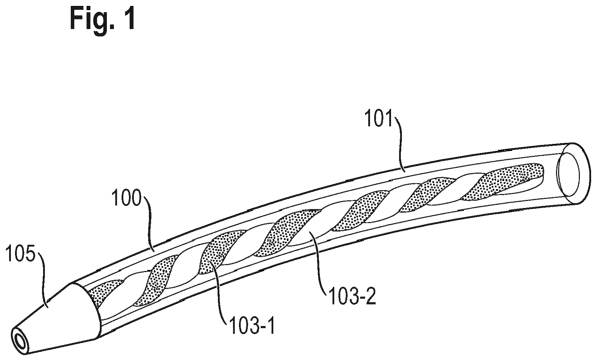

Fig. 1 zeigt eine schematische Darstellung eines Behälters 100 zum Erzeugen einer selbsthärtenden oder dualhärtenden zahnmedizinischen Kunststoffmasse. Innerhalb des schlauchförmigen Behälters 100 ist eine abgewogene Menge einer ersten Kunststoffkomponente 103-1 und eine abgewogene Menge einer zweiten Kunststoffkomponente 103-2 angeordnet.

Bei einem schlauchförmigen Behälter 100 lässt sich eine Beutelgröße, d.h. Länge des Schlauches, leicht an die zu mischende Materialmenge anpassen. Der schlauchförmige Behälter 100 weist beispielsweise einen Außendurchmesser von 5 bis 6 mm und eine Wandstärke von 1 mm auf. Das Volumen des Behälters 100 beträgt beispielsweise 1 ml.

Die beiden Kunststoffkomponenten 103-1 und 103-2 sind unvermischt in verschiedenen Volumenbereichen in dem Behälter 100 angeordnet. Die beiden Kunststoffkomponenten 103-1 und 103-2 werden in dem schlauchförmigen Behälter 100 angeordnet, der anschließend an einem oder beiden Enden verschlossen wird. Der Behälter 100 kann aus einem Schrumpfschlauch gebildet sein, der beim Befüllen mit den Kunststoffkomponenten 103-1 und 103-2 einen großen Durchmesser hat und dann durch Wärme deutlich kleiner wird. Dadurch kann Luft in dem Behälter 100 verdrängt werden, bevor dieser versiegelt und an den Enden zugeklemmt wird. Dadurch lassen sich Luftblasen in der Kunststoffmasse 200 vermeiden. Der Behälter 100 kann in einem Handgerät oder in einem stationären Gerät verwendet werden, das den Behälter 100 knetet und die Kunststoffmasse oder den gekneteten Behälter mit der Kunststoffmasse ausgibt.

Die Wandung 101 des Behälters 100 ist elastisch verformbar, so dass von außen eine mechanische Kraft auf die beiden Kunststoffkomponenten 103-1 und 103-2 ausgeübt werden kann. Beispielsweise kann die verformbare Wandung aus einem elastischen Silikon gebildet sein. Zum Vermischen der beiden Kunststoffkomponenten 103-1 und 103-2 wird der gesamte Behälter 100 geknetet. Dadurch vermischen sich die beiden Kunststoffkomponenten 103-1 und 103-2 und es entsteht eine homogene Kunststoffmasse im Inneren des Behälters 100, die anschließend verarbeitet werden kann. Die so gewonnene Kunststoffmasse dient beispielsweise zum Füllen eines Zahnes oder zum Befestigen einer Krone.

Die Kunststoffkomponenten 103-1 und 103-2 liegen strangförmig vor und sind spiralförmig miteinander verflochten. Dadurch kann eine gute Vermischung der beiden Kunststoffkomponenten 103-1 und 103-2 während des Knetvorgangs und eine homogene Kunststoffmasse erreicht werden. Die beiden Kunststoffkomponenten 103-1 und 103-2 können Pulver, Pasten oder Flüssigkeiten sein.

Die erste Kunststoffkomponente 103-1 umfasst beispielsweise eine polymerisierbare organische Matrix mit einer Mischung aus Monomeren, Initiator-Komponenten, Stabilisatoren und Pigmenten. Als Harze können Mischungen von Dimethacrylaten eingesetzt werden.

Bevorzugt sind Werkstoffe, die als radikalisch polymerisierbares Monomer (b) mindestens ein multifunktionelles (Meth)acrylat oder eine Mischung von mono- und multifunktionellen (Meth)acrylaten enthalten. Unter monofunktionellen (Meth)acrylaten werden Verbindungen mit einer, unter multifunktionellen (Meth)acrylaten Verbindungen mit zwei oder mehr, vorzugsweise 2 bis 4 radikalisch polymerisierbaren Gruppen verstanden. Die mono- oder multifunktionellen (Meth)acrylate können weitere funktionelle Gruppen, wie z.B. Hydroxy-, Ester- Silyl- oder Ureido-Gruppen enthalten. Geeignete mono- oder multifunktionelle (Meth)acrylate sind 2-Hydroxyethyl-, Benzyl-, Tetrahydrofurfuryl- oder Isobornylmethacrylat, p-Cumyl-phenoxyethylenglycolmethacrylat (CMP-1E), Bisphenol-A-dimethacrylat, Bis-GMA (ein Additionsprodukt aus Methacrylsäure und Bisphenol-A-diglycidylether), ethoxy- oder propoxyliertes Bisphenol-A-dimethacrylat, wie z.B. 2-[4-(2-(Meth)acryloyloxyethoxyethoxy)-phenyl]-2-[4-(2-methacryloyloxyethoxy)phenyl]-propan) (SR-348c, Firma Sartomer; enthält 3 Ethoxygruppen) oder 2,2-Bis[4-(2-methacryloxypropoxy)phenyl]propan (Bis-PMA), UDMA (ein Additionsprodukt aus 2-Hydroxyethylmethacrylat und 2,2,4- oder 2,4,4-Trimethylhexamethylen-1,6-diisocyanat), Di-, Tri- oder Tetraethylenglycoldimethacrylat, Trimethylolpropantrimethacrylat, Pentaerythrittetramethacrylat, sowie Glycerindi- und trimethacrylat, Bismethacryloyloxymethyltricyclo[5.2.1.]decan (TCDMA),1,4-Butandioldimethacrylat, 1,10-Decandioldimethacrylat (D3MA), 1,12-Dodecandioldimethacrylat oder Mischungen davon.

Besonders geeignet sind Bisphenol-A-dimethacrylat, Bis-GMA (ein Additionsprodukt aus Methacrylsäure und Bisphenol-A-diglycidylether), 2-[4-(2-(Meth)acryloyloxyethoxyethoxy)phenyl]-2-[4-(2-methacryloyloxyethoxy)phenyl]-propan) (SR-348c, Sartomer), 2,2-Bis[4-(2-methacryloxypropoxy)phenyl]propan (Bis-PMA), UDMA (ein Additionsprodukt aus 2-Hydroxyethylmethacrylat und 2,2,4- oder 2,4,4-Trimethylhexamethylen-1,6-diisocyanat), Bismethacryloyloxymethyltricyclo[5.2.1.]decan (TCDMA) und Mischungen davon.

Als zusätzliche Füllstoffe können anorganische partikuläre Füllstoffe, insbesondere Oxide, wie SiO2, ZrO2 und TiO2 oder Mischoxide aus SiO2, ZrO2, ZnO und/oder TiO2, nanopartikuläre oder mikrofeine Füllstoffe, wie pyrogene Kieselsäure oder Fällungskieselsäure, Glaspulver, wie Quarz-, Glaskeramik- oder röntgenopake Glaspulver, vorzugsweise Barium- oder Strontium-Aluminiumsilikatgläser, und röntgenopake Füllstoffe, wie Ytterbiumtrifluorid, Tantal(V)-oxid, Bariumsulfat oder Mischoxide von SiO2 mit Ytterbium(III)-oxid oder Tantal(V)-oxid verwendet werden.

Außerdem sind als Präpolymerisate (Isofüller) geeignet. Diese bestehen aus einer Monomermischung und Füllerkomponenten wie röntgenopake Glaspulver (Barium- oder Strontium-Aluminiumsilikatgläser), Oxide wie SiO2, ZrO2 und TiO2 und röntgenopaken Füllstoff (Ytterbiumtrifluorid). Diese Mischung wird zu Füllstoffen polymerisiert (thermisch, Licht) und ggf. nachbearbeitet (mahlen, sieben, silanisieren) und der Kunststoffmischung als Füllstoff zugesetzt.

Den Kunststoffkomponenten 103-1 und 103-2 sind Initiatoren zugesetzt, mit denen die Kunststoffkomponenten 103-1 und 103-2 selbst- oder dualhärtend polymerisiert werden können. Bei einer Selbsthärtung härtet die Kunststoffmasse allein durch die Mischung der beiden Kunststoffkomponenten 103-1 und 103-2 aus. Dabei werden die untern aufgeführten Initiatoren für eine bei Raumtemperatur durchgeführte selbsthärtende Polymerisation so zwischen den beiden Kunststoffkomponenten 103-1 und 103-2 aufgeteilt, dass die einzelnen Kunststoffkomponenten 103-1 und 103-2 stabil sind und erst bei Kontakt der beiden Kunststoffkomponenten 103-1 und 103-2 die Polymerisationsreaktion einsetzt. Bei einer Dualhärtung kann zusätzlich Licht zum Härten der Kunststoffmasse eingesetzt werden.

Bei einer Selbsthärtung härtet die Kunststoffmasse allein durch die Mischung der beiden Kunststoffkomponenten 103-1 und 103-2 aus. Bei einer Dualhärtung kann zusätzlich Licht zum Härten der Kunststoffmasse eingesetzt werden.

Als Initiatoren für eine bei Raumtemperatur durchgeführte selbsthärtende Polymerisation werden Redox-Initiatorkombinationen, wie z.B. Kombinationen von Benzoylperoxid mit N,N-Dimethyl-sym.-xylidin oder N,N-Dimethyl-p-toluidin, verwendet. Darüber hinaus sind auch Redoxsysteme bestehend aus Peroxiden bzw. Hydroperoxiden und solchen Reduktionsmitteln, wie z.B. Ascorbinsäure, Barbiturate, Thioharnstoffe oder Sulfinsäuren, besonders geeignet. Die Radikalbildungsgeschwindigkeit solcher Redoxinitiatorsysteme kann durch katalytische Mengen an Übergangsmetallverbindungen beschleunigt werden (Metallredoxkatalyse). Dabei lassen sich als Übergangsmetallredoxkatalysatoren Verbindungen von Übergangsmetallen, die mindestens 2-stabile Wertigkeitsstufen zeigen, einsetzen. Das sind vor allem Verbindungen der Elemente Kupfer, Eisen, Vanadium, Nickel oder Kobalt, wobei besonders bevorzugt Kupferverbindungen sind und diese bevorzugt als gut organolösliche Verbindungen, wie z.B. als Acetylacetonat, Naphthenat oder 2-Ethyl-hexanoat eingesetzt werden. Schließlich können auch für die Übergangsmetallverbindungen geeignete Liganden oder deren Komplexe als weitere Additive zugesetzt werden, insbesondere Chelatliganden, wie z.B. 2-(2-Aminoethylamino)ethanol, Triethylentetramin, Dimethylglyoxim, 8-Hydroxychinolin, 2,2'-Bipyridin oder 1,10-Phenanthrolin. Um eine ausreichende Lagerstabilität zu gewährleisten werden redoxinitiatorsystembasierende Materialien meist als 2-Komponenten-Systeme eingesetzt, wobei Oxydationsmittel (Peroxid- oder Hydroperoxid) und Reduktionsmittel (Amine, Sulfinsäuren, Barbiturate, Thioharnstoff etc.) in getrennten Komponenten eingearbeitet und erst kurz vor der Anwendung gemischt werden. Als Lichtinitiatoren für die Dualhärtung wird vorzugsweise eine Kombination mindestens eines monomolekularen Photoinitiators und mindestens eines bimolekularen Photoinitiators, insbesondere solche Photoinitiatoren, die in einem Wellenlängenbereich von 400 bis 500 nm aktiv sind. Bevorzugt werden der mindestens eine monomolekulare Photoinitiator und der mindestens eine bimolekulare Photoinitiator in einem Verhältnis von 2:1 bis 1:2, bezogen auf ihre Gewichtsanteile, eingesetzt.

Bevorzugte monomolekulare Photoinitiatoren sind Monoacyl- oder Bisacylphosphinoxide, Diacyldialkylgermanium- und Tetraacylgermanium-Verbindungen sowie Tetraacylstannane. Besonders bevorzugte monomolekulare Photoinitiatoren sind 2,4,6-Trimethylbenzoyldiphenylphosphinoxid, Bis(2,4,6-trimethylbenzoyl)phenyl-phosphinoxid, Dibenzoyldiethylgerman, Bis(4-methoxybenzoyl)diethylgerman (MBDEGe, Ivocerin®), Tetrabenzoylgerman, Tetrakis(o-methylbenzoyl)german, Tetrakis(mesitoyl)stannan und Mischungen davon.

Als bimolekulare Photoinitiatoren eigen sich vorzugsweise α-Diketone oder deren Derivate. Besonders geeignete α-Diketone oder Derivate davon sind Campherchinon (CQ), 9,10-Phenanthrenchinon, 1-Phenyl-propan-1,2-dion, 2,2-Dimethoxy-2-phenyl-acetophenon, Diacetyl oder 4,4'-Dichlorbenzil oder deren Derivate. Ganz besonders bevorzugt sind Campherchinon (CQ), 2,2-Dimethoxy-2-phenyl-acetophenon und Mischungen davon. Am meisten bevorzugt sind α-Diketone in Kombination mit Aminen als Reduktionsmittel, wie z.B. 4-(Dimethylamino)-benzoesäureester (EDMAB), N,N-Dimethylaminoethylmethacrylat, N,N-Dimethyl-sym.-xylidin, Triethanolamin und Mischungen davon. Vorzugsweise wird ein Verhältnis von bimolekular Photoinitiator zu Amin von 1:1 bis 1:6, bezogen auf ihre Gewichtsanteile, eingesetzt.

Durch den Behälter 100 wird der technische Vorteil erreicht, das die beiden Kunststoffkomponenten 103-1 und 103-2 bereits im richtigen Mischungsverhältnis im Behälter 100 vordosiert sein können und sich auch kleine Mengen im Bereich weniger Grammbruchteile der zahnmedizinischen Kunststoffmasse einfach und schnell herstellen lassen. Da das Mischen der beiden Kunststoffkomponenten 103-1 und 103-2 bei Bedarf in dem Behälter 100 erfolgt, entfällt ein Reinigungsaufwand und eine Kreuzkontamination kann verhindert werden. Ein Verkleben oder "Zupolymerisieren" der Kunststoffmasse in einer Mischmechanik kann verhindert werden.

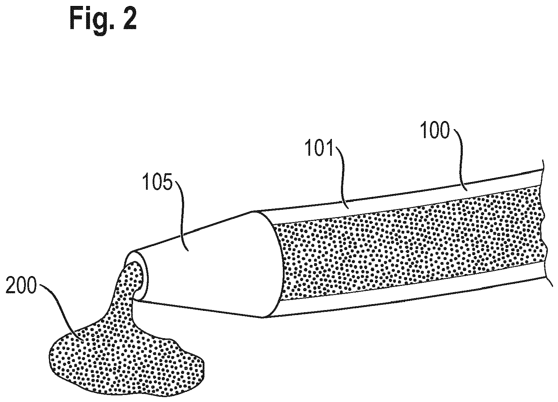

Fig. 2 zeigt eine weitere schematische Darstellung des Behälters 100 zum Erzeugen einer selbsthärtenden oder dualhärtenden zahnmedizinischen Kunststoffmasse 200 nach einem Knetvorgang. Die Kunststoffmasse 200 ist aus den beiden Kunststoffkomponenten 103-1 und 103-2 zusammengemischt und kann aus den Behälter 100 herausgedrückt werden.

Die Kunststoffmasse 200 bildet beispielsweise eine Paste für eine stopfbare Dentalformulierung mit einer Viskosität, die für fließfähige Kunststoffmassen im Bereich von als 50 - 3000 Pas, und vorzugsweise für stopfbare Kunststoffmassen zwischen 3000 - 50.000 Pas ist. Durch Energiezufuhr über eine Temperaturerhöhung, mittels Ultraschallanwendung oder durch Verdünnen mit einem Monomer kann eine Konsistenzanpassung erreicht werden.

Wird die Kunststoffmasse 200 aus dem schlauchförmigen Behälter 100 herausgedrückt, z.B. von Hand, mit einem Kolben oder mit einer Walze, ist die Kunststoffmasse 200 homogen gemischt. Die Kunststoffmasse 200 kann selbst- oder dualhärtend sein und vollständig aus dem Behälter 100 ausgebracht werden.

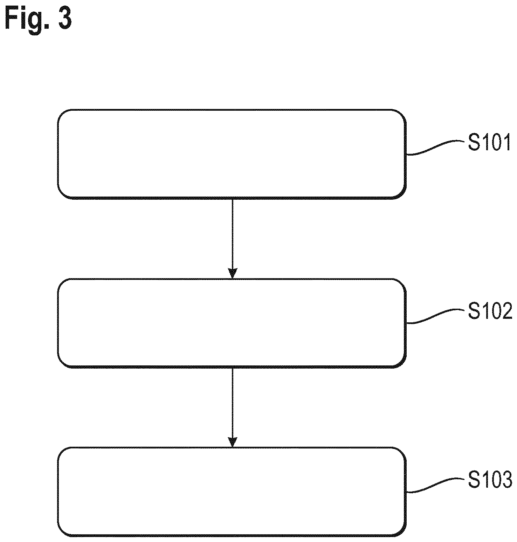

Fig. 3 zeigt ein Blockdiagramm eines Verfahrens zum Erzeugen einer selbsthärtenden, zahnmedizinischen Kunststoffmasse 200. Die beiden Kunststoffkomponenten 103-1 und 103-2 befinden sich im Inneren des Behälters 100 in einem unvermischten Zustand, in dem die erste Kunststoffkomponente 103-1 und die zweite Kunststoffkomponente 103-2 in unterschiedlichen räumlichen Volumenbereichen des Behälters 100 vorliegen.

Im ersten Schritt S101 kann der Behälter 100 zunächst auf eine vorgegebene Temperatur erwärmt werden. Dadurch lässt sich die Durchmischung der beiden Kunststoffkomponente 103-1 und 103-2 beschleunigen und verbessern. Durch das Erwärmen lassen sich Mischdauern im Minutenbereich erzielen. Beispielsweise wird der verschlossene schlauchförmige Behälter 100 in einem Heizschrank auf 80°C erwärmt und anschließend auf einer ebenfalls 80°C warmen Heizplatte von Hand mit etwas Druck gewalzt.

Es kann auch ein Teil einer Mischeinheit mit einem Rotor und einem Stator oder die ganze Mischeinheit erwärmt werden, um so das Material aufzuheizen. Das Dosieren des Materials, das Mischen und das Erwärmen kann in einem Gerät ohne manuelle Zwischenschritte erfolgen.

Anschließend wird in Schritt S102 der Behälter 100 mit der verformbaren Wandung 101 mechanisch geknetet, um eine homogene Vermischung der beiden Kunststoffkomponenten 103-1 und 103-2 in Inneren des Behälters 100 zu erreichen. Das Kneten kann ein Walken, Rollen, Walzen oder Drücken des Behälters 100 umfassen.

In Schritt S103 wird die so aus den beiden Kunststoffkomponenten 103-1 und 103-2 erzeugte Kunststoffmasse 200 aus dem Behälter 100 herausgedrückt, damit diese ihrem Zweck entsprechend verarbeitet werden kann, wie beispielsweise als Zahnfüllung oder als Kleber. Durch die Vermischung der Kunststoffkomponenten 103-1 und 103-2 härtet die Kunststoffmasse 200 aus. Durch das Verfahren wird der technische Vorteil erreicht, dass sich auch geringe Mengen der Kunststoffmasse 200 mit einem präzisen Mischungsverhältnis bei der zahnmedizinischen Anwendung auf schnelle und einfache Weise herstellen lassen.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Behälter
- 101: Wandung
- 103-1: Kunststoffkomponente
- 103-2: Kunststoffkomponente
- 105: Tülle
- 200: Kunststoffmasse

## Patentansprüche

1. Behälter (100) zum Erzeugen einer selbsthärtenden oder dualhärtenden zahnmedizinischen Kunststoffmasse (200), der eine verformbare Wandung (101) aufweist, mit:
einer ersten Kunststoffkomponente (103-1); und
einer zweiten Kunststoffkomponente (103-2) zum Härten der Kunststoffmasse (200) oder Starten einer Härtungsreaktion.

2. Behälter (100) nach Anspruch 1, wobei der Behälter (100) schlauchförmig oder beutelförmig ist.

3. Behälter (100) nach einem der vorangehenden Ansprüche, wobei die verformbare Wandung (101) aus Silikon gebildet ist.

4. Behälter (100) nach einem der vorangehenden Ansprüche, wobei die verformbare Wandung (101) in einem Wellenlängenbereich von 390 nm bis 520 nm transparent ist.

5. Behälter (100) nach einem der vorangehenden Ansprüche, wobei die erste Kunststoffkomponente (103-1) und/oder die zweite Kunststoffkomponente (103-2) strangförmig innerhalb des Behälters (100) angeordnet sind.

6. Behälter (100) nach Anspruch 5, wobei die strangförmige, erste Kunststoffkomponente (103-1) und die strangförmige, zweite Kunststoffkomponente (103-2) miteinander verflochten sind.

7. Behälter (100) nach einem der vorangehenden Ansprüche, wobei die erste Kunststoffkomponente (103-1) eine polymerisierbare organische Matrix mit einer Mischung aus Monomeren, Initiator-Komponenten, Stabilisatoren und Pigmenten umfasst.

8. Behälter (100) nach einem der vorangehenden Ansprüche, wobei die erste und/oder zweite Kunststoffkomponente (103-1, 103-2) pulverförmig, pastenartig oder flüssig ist.

9. Behälter (100) nach einem der vorangehenden Ansprüche, wobei die Kunststoffmasse (200) aus der vermischten ersten Kunststoffkomponente (103-1) und zweiten Kunststoffkomponente (103-2) eine Viskosität aufweist, die für fließfähige Kunststoffmassen im Bereich von als 50 - 3000 Pas, und vorzugsweise für stopfbare Kunststoffmassen zwischen 3000 - 50.000 Pas ist.

10. Behälter (100) nach einem der vorangehenden Ansprüche, wobei der Behälter (100) eine Tülle (105) zum Ausdrücken der Kunststoffmasse (200) umfasst.

11. Verfahren zum Erzeugen einer selbsthärtenden oder dualhärtenden zahnmedizinischen Kunststoffmasse (200), mit dem Schritt:
Kneten (S101) eines Behälters (100) mit einer verformbaren Wandung (101), der eine erste Kunststoffkomponente (103-1) und eine zweite Kunststoffkomponente (103-2) zum Härten der Kunststoffmasse (200) oder Starten einer Härtungsreaktion umfasst.

12. Verfahren nach Anspruch 11, wobei der Behälter (100) vor oder während dem Schritt (S101) des Knetens auf eine vorgegebene Temperatur erwärmt wird.

13. Verfahren nach Anspruch 11 oder 12, wobei das Kneten oder Mischen der ersten und zweiten Kunststoffkomponente (103-1, 103-2) bei einem Unterdruck oder unter Vakuum durchgeführt wird.
